(19) Europäisches Patentamt
European Patent Office
Office européen des brevets

(11) **EP 2 760 361 B1**

(12) **FASCICULE DE BREVET EUROPEEN**

(45) Date de publication et mention de la délivrance du brevet:
**15.11.2017 Bulletin 2017/46**

(21) Numéro de dépôt: **12766104.9**

(22) Date de dépôt: **28.09.2012**

(51) Int Cl.:
*A61B 34/00* *(2016.01)*     *A61B 90/50* *(2016.01)*

(86) Numéro de dépôt international:
**PCT/EP2012/069236**

(87) Numéro de publication internationale:
**WO 2013/045645 (04.04.2013 Gazette 2013/14)**

(54) **DISPOSITIF DE GUIDAGE D'UN INSTRUMENT MEDICAL INSERE DANS UNE VOIE NATURELLE OU UNE VOIE ARTIFICIELLE D'UN PATIENT**

VORRICHTUNG ZUR FÜHRUNG EINES IN EINEN NATÜRLICHEN ODER KÜNSTLICHEN KANAL IM KÖRPER EINES PATIENTEN EINGESETZTEN MEDIZINISCHEN INSTRUMENTS

DEVICE FOR GUIDING A MEDICAL INSTRUMENT INSERTED INTO A NATURAL DUCT OR AN ARTIFICIAL DUCT OF A PATIENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2011 FR 1158880**

(43) Date de publication de la demande:
**06.08.2014 Bulletin 2014/32**

(73) Titulaires:
• **Université Pierre et Marie Curie (Paris 6)**
**75005 Paris (FR)**
• **Koelis**
**38240 Meylan (FR)**
• **Centre National de la Recherche Scientifique**
**75016 Paris (FR)**

(72) Inventeurs:
• **POQUET, Cécile**
**F-94200 Ivry-sur-seine (FR)**
• **MOZER, Pierre**
**F-94300 Vincennes (FR)**
• **BAUMANN, Michael**
**F-38100 Grenoble (FR)**
• **VITRANI, Marie-Aude**
**F-75013 Paris (FR)**
• **MOREL, Guillaume**
**F-75013 Paris (FR)**
• **LEROY, Antoine**
**F-38240 Meylan (FR)**
• **HENRI, Patrick**
**F-92270 Bois-Colombes (FR)**

(74) Mandataire: **Decorchemont, Audrey Véronique Christèle**
**CABINET BOETTCHER**
**16, rue Médéric**
**75017 Paris (FR)**

(56) Documents cités:
**EP-A2- 1 520 548          WO-A1-2011/058530**
**DE-A1-102006 007 858     US-A1- 2002 177 857**
**US-A1- 2004 092 810       US-A1- 2009 000 627**

## Description

L'invention concerne un dispositif de guidage d'un instrument médical inséré dans une voie naturelle ou une voie artificielle d'un patient afin d'amener au moins une extrémité distale de l'instrument à proximité d'un organe interne.

ARRIERE PLAN TECHNOLOGIQUE DE L'INVENTION

Les opérations dites « ouvertes » s'avèrent très lourdes pour un patient. Ainsi, de plus en plus, les praticiens ont recours à des opérations mini-invasives lors desquelles des instruments médicaux sont insérés dans une voie naturelle du patient (vagin, rectum, conduit auditif...) ou dans une voie artificielle rapportée sur le corps du patient (canule, veine artificielle, trocart...).

En urologie, afin de dépister un éventuel cancer de la prostate, il est connu de réaliser une biopsie prostatique. Il s'agit de prélever des échantillons de tissus au sein même de la prostate, lesdits échantillons étant ensuite analysés en laboratoire afin de détecter la présence d'éventuelles cellules cancéreuses. A cet effet, le patient est couché sur le flanc. Un instrument, comportant une sonde échographique et un porte-aiguille portant une aiguille à biopsie, est alors inséré dans la voie naturelle qu'est le rectum. Grâce à l'instrument, le chirurgien perce la paroi du colon pour atteindre la prostate et prélever ainsi des échantillons de tissus.

Pour réaliser la biopsie, le chirurgien peut seulement s'aider des images en deux dimensions prises en temps réel par la sonde échographique. Le chirurgien doit ainsi estimer de tête une représentation en trois dimensions de la prostate pour réaliser les prélèvements d'échantillons, sachant que la prostate à tendance à se déplacer voir à se déformer du fait des mouvements de l'instrument.

Il s'avère donc extrêmement difficile pour un chirurgien de connaître précisément en quels endroits l'aiguille va venir prélever des échantillons de tissus prostatique. Il convient pourtant de réaliser des ponctions de la prostate de façon uniforme sur toute la prostate, les cellules cancéreuses n'étant pas directement détectables par la sonde.

Récemment, des dispositifs sont apparus afin d'aider le chirurgien à réaliser très précisément les différentes ponctions. En effet, ces nouveaux dispositifs comportent des sondes permettant de fournir des images en trois dimensions de la prostate. Le principe de fonctionnement desdites sondes est de réaliser un planning de positions de ponctions théoriques et d'étudier au cours de la biopsie la déformation de la prostate. Le planning de ponctions théoriques est alors modifié en fonction de l'étude de la déformé de la prostate afin de réaliser les ponctions aux bons endroits.

Toutefois, les sondes doivent scanner la prostate pour fournir des images en trois dimensions et fournissent ainsi des images non pas en temps réel mais avec décalage de quelques secondes. Il convient donc de limiter au maximum tout mouvement de la sonde lorsque celle-ci scanne la prostate. De la même façon, il convient de limiter au maximum tout mouvement parasite de l'instrument lorsque l'aiguille à biopsie est injectée dans la prostate afin de réaliser la ponction au bon endroit.

Or il se peut que le patient ne soit pas sous anesthésie locale et/ou qu'il ne soit pas immobilisé. Ainsi, lors de l'intervention, le patient peut bouger et avec lui tous les tissus de la voie naturelle ou artificielle avec lesquels l'instrument peut entrer en contact. En outre, il s'avère difficile pour un chirurgien de maintenir constamment l'instrument dans une position donnée pour permettre à la sonde de scanner la prostate et réaliser les ponctions aux endroits voulus.

Afin d'aider le chirurgien, il est connu des dispositifs de guidage d'instrument comportant un bras articulé pour déplacer une extrémité proximale de l'instrument. Le chirurgien peut alors commander le bras articulé pour que celui-ci bloque l'instrument dans une position donnée. Le chirurgien peut ainsi effectuer aisément une opération sur le patient comme par exemple le prélèvement d'un échantillon de tissu ou bien l'acquisition d'images par la sonde.

Toutefois, l'instrument ainsi bloqué dans une position donné s'oppose aux mouvements du patient ce qui peut incommoder le patient voir endommager une partie de ses tissus.

Il est ainsi connu des dispositifs de guidage d'instrument comportant un bras articulé pour déplacer une extrémité proximale de l'instrument, le bras articulé étant lié à l'extrémité proximale par une liaison de type à cardan ou à rotule.

Ainsi, même si le chirurgien commande le bras articulé pour que celui-ci bloque l'instrument dans une position donnée, la liaison rotule ou cardan autorise malgré tout des mouvements du patient. Mais à chaque mouvement du patient, l'instrument se trouve déplacé et le chirurgien doit alors replacer correctement l'instrument.

Il est connu du document WO 2011/058530 un dispositif de guide d'instrument médical comportant un bras articulé pouvant bloquer l'instrument médical dans une position donnée. Le bras articulé est commandé de sorte que lorsque l'effort exercé par le chirurgien sur l'instrument dépasse un seuil d'effort prédéterminé, le blocage du bras articulé est forcé. L'effort exercé est cependant mesuré par des capteurs d'effort.

OBJET DE L'INVENTION

Un but de l'invention est de proposer un dispositif de guidage d'un instrument médical inséré dans une voie naturelle ou une voie artificielle d'un patient plus perfectionné que les dispositifs existants.

BREVE DESCRIPTION DE L'INVENTION

En vue de la réalisation de ce but, on propose un dispositif de guidage d'un instrument médical inséré dans une voie naturelle ou une voie artificielle d'un patient afin d'amener au moins une extrémité distale de l'instrument à proximité d'un organe interne le dispositif comportant un bras articulé comportant une pluralité de degrés de liberté pour déplacer une extrémité proximale de l'instrument, des moyens de blocage commandés des degrés de liberté du bras articulé, les moyens de blocage étant agencés de sorte que, pour au moins l'un des degrés de liberté, un blocage imposé audit degré de liberté puisse être forcé lorsqu'un effort externe exercé sur l'instrument dépasse un seuil prédéterminé, et un module de calcul pour commander les moyens de blocage.

Selon l'invention le dispositif comporte des moyens d'acquisition d'images de l'organe qui sont portés par l'instrument, le module de calcul se basant au moins sur lesdites images pour commander les moyens de blocage.

Ainsi, l'instrument est immobilisé dans une position donnée afin de permettre au chirurgien de pratiquer aisément une opération sur le patient (scanner de l'organe, biopsie de l'organe...) mais le blocage peut être forcé de sorte que si l'effort externe est dû à un mouvement brusque du patient, l'anatomie du patient est bien protégée. En revanche, de petits mouvements du patient ne viennent pas perturber le travail du chirurgien.

Grâce à la commande des moyens de blocage à partir des images de l'organe, il est possible de débloquer le bras articulé non seulement lorsque l'effort externe est dû au mouvement d'un chirurgien agissant sur l'organe du patient mais également lorsque l'effort externe est dû au mouvement du patient par exemple lorsque ce dernier éternue. L'anatomie du patient est ainsi bien mieux protégée.

Par 'forcer le blocage', on entend ici que lorsqu'un effort externe dépasse un seuil prédéterminé, les moyens de blocage sont agencés pour rendre de nouveau mobile le bras articulé selon le degré de liberté qui était initialement bloqué, l'instrument pouvant alors se déplacer en réponse à l'effort externe s'exerçant sur lui.

De préférence, le module de calcul comporte des moyens de mémorisation d'une position à atteindre par l'instrument relativement à l'organe, le module de calcul commandant, à partir des images prises par les moyens d'acquisition d'images, les moyens de blocage pour imposer un blocage à au moins l'un des degrés de liberté du bras articulé lorsque l'instrument est sensiblement proche de ladite position.

Grâce à ce contrôle, il est possible de bloquer le bras articulé dans une position recherchée précise ce qui facilite le travail du chirurgien.

BREVE DESCRIPTION DES DESSINS

L'invention sera mieux comprise à la lumière de la description qui suit de modes de réalisation non limitatifs de l'invention en référence aux figures ci-jointes parmi lesquelles :

- la figure 1 est une vue schématique d'un dispositif de guidage selon l'invention, une partie dudit dispositif de guidage étant représentée insérée dans une voie du patient;
- la figure 2 est une vue schématique d'un dispositif de guidage selon un mode de réalisation particulier de l'invention, une partie dudit dispositif de guidage étant représentée inséré dans le rectum d'un patient ;
- la figure 3 est un schéma illustrant les différentes étapes d'un premier mode de mise en oeuvre du dispositif illustré à la figure 1 ;
- la figure 4 est un schéma illustrant les différentes étapes d'un deuxième mode de mise en oeuvre du dispositif illustré à la figure 1 ;
- la figure 5 est un schéma illustrant les différentes étapes d'un troisième mode de mise en oeuvre du dispositif illustré à la figure 1.

DESCRIPTION DETAILLEE DE L'INVENTION

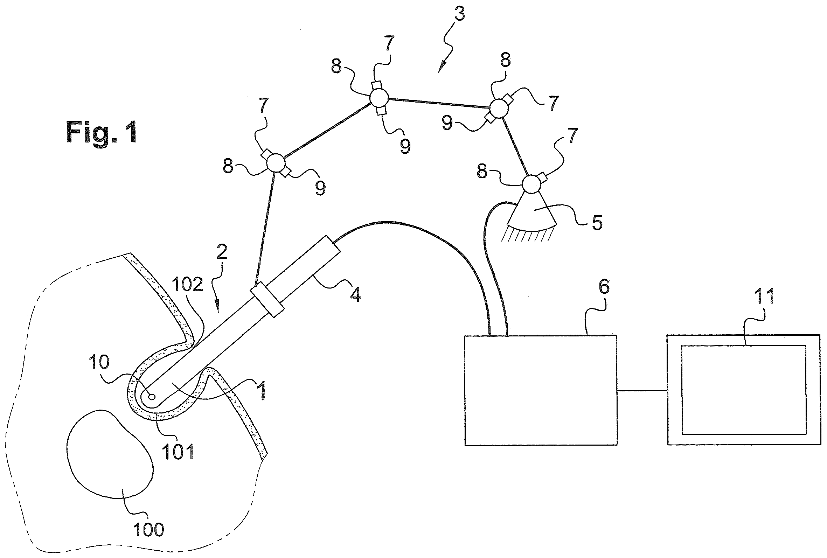

En référence à la figure 1, le dispositif de guidage selon l'invention est destiné à amener au moins une extrémité distale 1 d'un instrument médical 2 à proximité d'un organe interne 100 d'un patient. L'instrument médical 2 est par exemple une sonde échographique, un porte-aiguille... L'instrument médical 2 est inséré dans une voie 101 du patient, qui peut être naturelle (rectum, vagin, conduit auditif ...) ou artificielle (canule, trocart, veine artificielle ...), à travers un orifice 102 du patient, qui peut être naturel (anus ....) ou artificiel (incision ...).

Le dispositif de guidage comporte un bras articulé 3 comportant une pluralité de degrés de liberté pour déplacer une extrémité proximale 4 de l'instrument. Par extrémité proximale, on entend bien entendu l'extrémité opposée à celle introduite dans le corps du patient. Le bras articulé 3 est ici relié à l'une de ses extrémités à une base 5 et porte à l'autre de ses extrémités l'instrument médical 2.

Le dispositif de guidage comporte en outre des moyens de blocage commandés des degrés de liberté du bras articulé 3 afin de bloquer le bras articulé 3 dans une position donnée. Les moyens de blocage facilitent ainsi le positionnement de l'instrument médical 2 et son maintien dans une position particulière. Les moyens de blocage comportent par exemple au moins un moteur monté sur une articulation du bras articulé 3, au moins un organe de freinage agencé sur une articulation du bras articulé 3 tel qu'un frein électromagnétique, un frein à friction ..., au moins un embrayage à dent ou à crabot monté sur une articulation du bras articulé 3...

Les moyens de blocage sont agencés de sorte que, pour au moins l'un des degrés de liberté du bras articulé 3, un blocage imposé audit degré de liberté puisse être forcé lorsqu'un effort externe exercé sur l'instrument médical 2 dépasse un seuil prédéterminé. L'effort externe est par exemple un mouvement du patient ou un mouvement d'un chirurgien agissant sur l'organe du patient. Le bras articulé est alors de nouveau déplaçable au niveau du degré de liberté non bloqué de sorte que l'instrument puisse se déplacer en réponse à l'effort externe s'exerçant sur lui. De façon avantageuse, le mouvement imposé à l'instrument par l'effort externe est autorisé mais demeure freiné par les moyens de blocage. Selon un mode de réalisation privilégié, les moyens de blocage sont agencés de sorte que plus l'effort externe est important moins les moyens de blocage freinent le mouvement imposé à l'instrument par l'effort externe. De préférence, le dispositif de guidage est agencé de sorte que lorsque le blocage est forcé, aucune action ne puisse être effectuée sur l'organe 100 par l'instrument 2.

Les moyens de blocage sont par exemple agencés de sorte que le seuil prédéterminé soit défini directement par le chirurgien avant le début d'un examen ou au cours de l'examen. Selon une variante, les moyens de blocage sont agencés de sorte que le seuil prédéterminé soit déterminé en fonction des efforts externes déjà exercés sur l'instrument 2 lorsque les moyens de blocage viennent imposer un blocage des degrés de liberté du bras articulé 3. Ainsi, si l'instrument 2 est bloqué dans une position pour laquelle des efforts externes sont déjà exercés sur l'instrument 2, le blocage ne sera pas forcé au moindre effort externe supplémentaire, le seuil prédéterminé étant défini relativement aux efforts externes initialement exercés sur l'instrument 2.

Selon l'invention, les moyens de blocage sont commandés par un organe de calcul 6 du dispositif de guidage encore appelé module de calcul 6 du dispositif de commande. Par exemple, lorsque les moyens de blocage comportent au moins un moteur monté sur une articulation du bras articulé 3, le module de calcul 6 module un courant électrique traversant le moteur pour venir forcer électroniquement le blocage des moyens de blocage.

Selon l'invention, le dispositif comporte des moyens d'acquisition d'images 10 de l'organe 100 qui sont portés par l'instrument médical 2. L'instrument médical 2 comporte ainsi les moyens d'acquisition d'images 10 de l'organe 100 et est lié auxdits moyens d'acquisition d'images 10. Les images prises par lesdits moyens d'acquisition d'images sont ainsi représentatives de la position desdits moyens vis-à-vis de l'organe 100 dans le référentiel du dispositif de guidage.

Selon l'invention, le module de calcul 6 se base sur les images prises par les moyens d'acquisition d'images 10 pour commander les moyens de blocage. Selon un mode de réalisation privilégié, le module de calcul 6 comporte des moyens d'estimation d'un déplacement de l'organe 100 relativement à l'instrument 2 et/ou d'une déformée de l'organe 100 à partir des images fournies par les moyens d'acquisition d'images 10 lorsque l'instrument 2 est au contact de l'organe. Le module de calcul 6 comporte en outre des moyens de contrôle communiquant avec les moyens d'estimation pour déterminer à partir de l'estimation du déplacement et/ou de la déformée si le blocage doit être forcé.

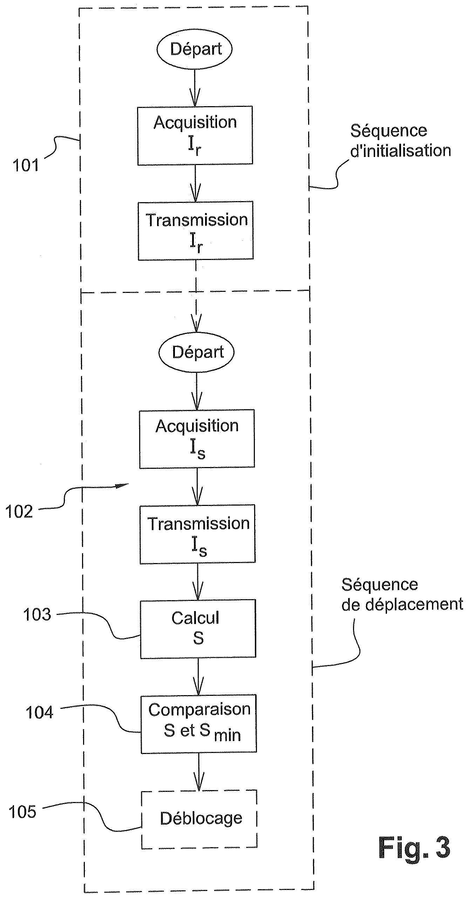

En référence à la figure 3, selon un premier mode de mise en oeuvre, le module de calcul 6 commande un déblocage des moyens de blocage comme suit.

Lors d'une séquence d'initialisation, au cours d'une première étape 101, les moyens d'acquisition 10 acquièrent une première image dite image de référence $I_r$. Ladite image de référence est ensuite transmise au module de calcul 6. L'image de référence est par exemple une image générale de l'organe 100 ou bien une image d'une zone particulière de l'organe 100. L'image de référence est par exemple en deux dimensions ou en trois dimensions. En variante, l'image de référence n'est pas acquise par les moyens d'acquisition 10 mais est acquise par d'autres analyses médicales (comme une Imagerie par Résonnance Magnétique) et fournie au module de calcul 6.

Lors d'une séquence de déplacement de l'instrument 2, au cours d'une deuxième étape 102, les moyens d'acquisition 10 acquièrent au moins une image $I_s$ et la transmettent au module de calcul 6.

Lors d'une troisième étape 103, les moyens d'estimation évaluent un paramètre de similarité S entre l'image de référence $I_r$ et l'image $I_s$. Une telle évaluation est par exemple réalisée selon la formule:

$$S = Sim(I_s, I_r \circ T_m)$$

où, Sim est une fonction de mesure de la corrélation croisée de deux images (ou de deux sous-ensembles d'images) qui est déterminée sur les niveaux de gris des images (ou des éléments des deux sous-ensembles d'images).

Lors d'une quatrième étape 104, les moyens d'estimation comparent le paramètre de similarité S à un seuil de similarité prédéterminé $S_{min}$ qui est représentatif du seuil d'effort prédéterminé des moyens de blocage. Ledit seuil de similarité $S_{min}$ est par exemple calculé à partir d'analyses statistiques de données de plusieurs tests initiaux comme un algorithme d'apprentissage bayésien.

Lors d'une cinquième étape 105, les moyens de contrôle commandent les moyens de blocage pour forcer le blocage imposé si le paramètre de similarité S est inférieur au seuil de similarité prédéterminé $S_{min}$.

Si l'organe 100 s'est déplacé ou déformé entre l'image de référence $I_r$ et l'image $I_s$ cela signifie que l'instrument 2 s'est déplacé relativement à l'organe 100 et est éventuellement au contact de l'organe 100, le déplacement de l'instrument 2 étant dû à un effort externe provoqué par un mouvement du patient ou à un mouvement du chirurgien. Le module de calcul 6 estime ainsi les efforts externes exercés sur l'instrument 2 à partir des images acquises par les moyens d'acquisition 10 en calculant les mouvements de l'instrument 2 par rapport à l'organe 100.

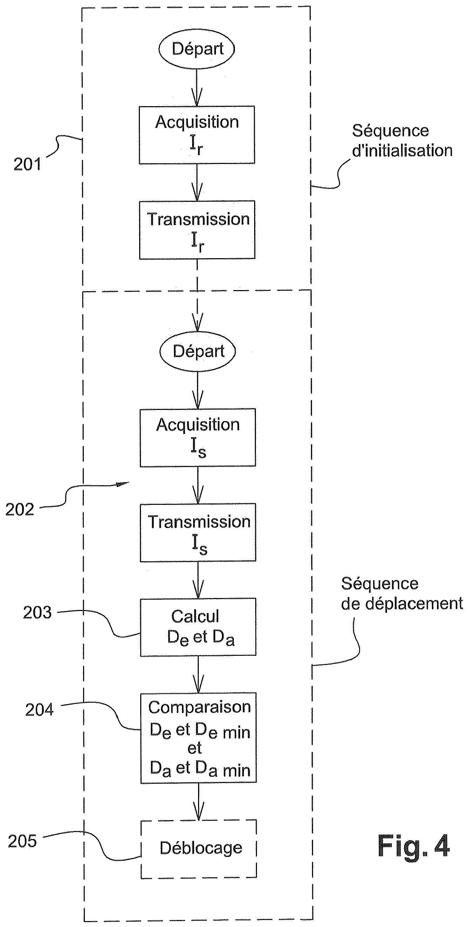

En référence à la figure 4, selon un deuxième mode de mise en oeuvre, le module de calcul 6 commande un déblocage des moyens de blocage comme suit.

Lors d'une séquence d'initialisation, au cours d'une première étape 201, les moyens d'acquisition 10 acquièrent une première image dite image de référence $I_r$. Ladite image de référence est ensuite transmisse au module de calcul 6. En variante, l'image de référence $I_r$ n'est pas acquise par les moyens d'acquisition 10 mais est acquise par d'autres analyses médicales et fournie au module de calcul 6. Dans tous les cas, il est préférable mais non limitatif dans ce mode de mise en oeuvre que l'image de référence $I_r$ soit une image générale de l'organe 100 en trois dimensions.

Lors d'une séquence de déplacement de l'instrument 2, au cours d'une deuxième étape 202, les moyens d'acquisition 10 acquièrent au moins une image $I_s$ et la transmettent au module de calcul 6.

Lors d'une troisième étape 203, les moyens d'estimation évaluent un paramètre de distance euclidienne De entre l'image de référence $I_r$ et l'image $I_s$ et un paramètre de distance angulaire $D_a$ entre l'image de référence $I_r$ et l'image $I_s$. Une telle évaluation est par exemple réalisée à partir d'un recalage entre les deux images ou encore en recherchant une fonction qui minimise un paramètre de similarité entre les deux images par optimisation par la méthode de Powell-Brent.

Lors d'une quatrième étape 204, les moyens d'estimation comparent le paramètre de distance euclidienne De à un seuil de distance euclidienne prédéterminé $De_{min}$ qui est représentatif du seuil d'effort prédéterminé des moyens de blocage. Les moyens d'estimation comparent en outre le paramètre de distance angulaire $D_a$ à un seuil de distance angulaire prédéterminé $Da_{min}$ qui est représentatif du seuil d'effort prédéterminé des moyens de blocage. Lesdits seuils $De_{min}$ et $Da_{min}$ sont par exemple calculés à partir d'une marge d'erreur acceptable entre la position souhaitée de l'instrument 2 et la position réelle de l'instrument 2.

Lors d'une cinquième étape 205, les moyens de contrôle commandent les moyens de blocage pour forcer le blocage imposé si le paramètre de distance euclidienne $D_e$ dépasse le seuil de distance euclidienne prédéterminé $De_{min}$ ou si le paramètre de distance angulaire $D_a$ dépasse le seuil de distance angulaire prédéterminé $Da_{min}$.

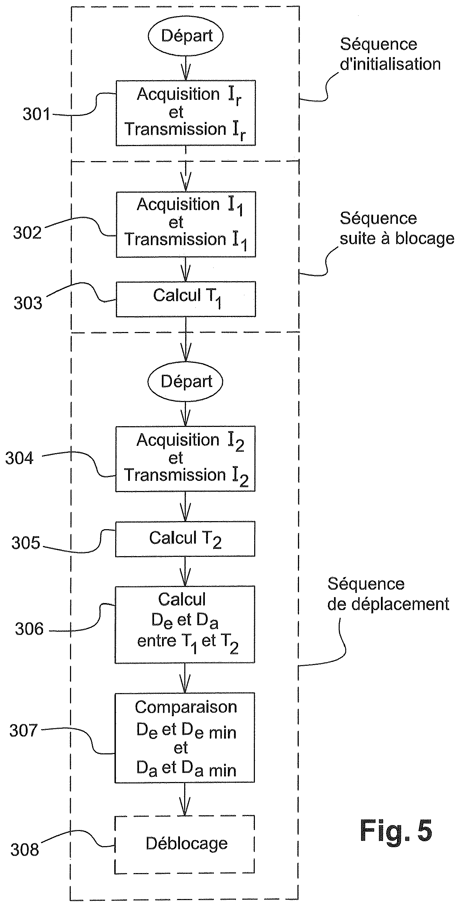

En référence à la figure 5, selon un troisième mode de mise en oeuvre, le module de calcul 6 commande un déblocage des moyens de blocage comme suit.

Lors d'une séquence d'initialisation, au cours d'une première étape 301, les moyens d'acquisition 10 acquièrent une première image dite image de référence $I_r$. Ladite image de référence est ensuite transmisse au module de calcul 6.

Suite au blocage des moyens de blocage, au cours d'une deuxième étape 302, les moyens d'acquisition 10 acquièrent au moins une première image $I_1$ et la transmettent au module de calcul 6.

Lors d'une troisième étape 303, les moyens d'estimation évaluent une première position $T_1$ de la première image $I_1$ relativement à l'image de référence $I_r$.

Lors d'une séquence de déplacement de l'instrument 2, au cours d'une quatrième étape 304, les moyens d'acquisition 10 acquièrent au moins une deuxième image $I_2$ et la transmettent au module de calcul 6.

Lors d'une cinquième étape 305, les moyens d'estimation évaluent une deuxième position $T_2$ de la deuxième image $I_2$ relativement à l'image de référence $I_r$.

Lors d'une sixième étape 306, les moyens d'estimation évaluent un paramètre de distance euclidienne De entre la première position $T_1$ et la deuxième position $T_2$ et un paramètre de distance angulaire $D_a$ entre la première position $T_1$ et la deuxième position $T_2$.

Lors d'une septième étape 307, les moyens d'estimation comparent le paramètre de distance euclidienne $D_e$ à un seuil de distance euclidienne prédéterminé $De_{min}$ qui est représentatif du seuil d'effort prédéterminé des moyens de blocage. Les moyens d'estimation comparent en outre le paramètre de distance angulaire $D_a$ à un seuil de distance agualaire prédéterminé $Da_{min}$ qui est représentatif du seuil d'effort prédéterminé des moyens de blocage.

Lors d'une huitième étape 308, les moyens de contrôle commandent les moyens de blocage pour forcer le blocage imposé si le paramètre de distance euclidienne De est inférieur au seuil de distance euclidienne prédéterminé $De_{min}$ ou si le paramètre de distance angulaire $D_a$ est inférieur au seuil de distance angulaire prédéterminé $Da_{min}$.

De façon particulière, pour commander les moyens de blocage, le module de calcul 6 détermine en outre la valeur de l'effort externe appliqué sur l'instrument 2 à l'aide de capteurs d'effort. Selon une variante, un capteur, par exemple un capteur angulaire 7, est agencé sur chaque articulation 8 du bras articulé 3 pour transmettre au module de calcul 6 un signal représentatif de la position relative de deux éléments du bras articulé 3 formant l'articulation 8 associé. De façon avantageuse, grâce aux signaux des capteurs angulaires 7, le module de calcul 6 peut estimer la position de l'instrument 2 dans un référentiel du dispositif de guidage. Cette estimation peut par exemple être transmise au chirurgien pour l'aider à visualiser la position de l'instrument 2 vis-à-vis de l'organe 100.

De façon privilégiée, le dispositif de guidage comporte différents modes d'utilisation. Selon un premier mode d'utilisation du dispositif de guidage, les moyens de blocage sont désactivés de sorte qu'aucun degré de liberté du bras articulé 3 n'est bloqué. Le bras articulé 3 peut donc être librement déplacé de façon automatique et/ou de façon manuelle par le chirurgien. Dans un deuxième mode d'utilisation, les moyens de blocage sont activés de sorte que les degrés de liberté du bras articulé 3 soient bloqués. L'extrémité proximale 1 de l'instrument 2 est donc bloquée dans une position donnée.

Lors d'une intervention, le chirurgien peut ainsi basculer autant de fois que nécessaire entre les deux modes d'utilisation du dispositif de guidage. Par exemple, le chirurgien peut sélectionner dans un premier temps le premier mode d'utilisation pour placer librement l'extrémité distale 1 de l'instrument 2 dans la voie naturelle 101 du patient. Puis, dans un deuxième temps, le chirurgien sélectionne le deuxième mode d'utilisation pour maintenir l'instrument dans une position donnée.

De préférence, le dispositif de guidage comporte des moyens de sélection des modes d'utilisation du dispositif de guidage. Les moyens de sélection comporte par exemple un ou des interrupteurs agencés par exemple sur le module de calcul 6 et/ou sur le bras articulé 3. En variante, les moyens de sélection comportent une ou des pédales agencées au pied du dispositif de guidage, un système de commande vocale ... De préférence, les moyens de sélection sont agencés de sorte que le chirurgien puisse basculer d'un mode d'utilisation à un autre de façon instantanée et à tout moment de l'intervention.

De préférence, et de façon connue en soi, le dispositif de guidage comporte des compensateurs de poids 9 pour équilibrer le bras articulé 3 de sorte que l'extrémité proximale 4 de l'instrument 2 soit maintenue dans une position particulière, préférentiellement au centre de la voie 101. Ainsi, lors du passage du deuxième mode d'utilisation au premier mode d'utilisation, l'instrument 2 reste sensiblement au centre de l'orifice 102.

De préférence, le dispositif de guidage comporte des moyens de communication avec le chirurgien pour, par exemple, indiquer la position de l'instrument 2 par rapport à l'organe 100 afin d'aider le chirurgien dans une planification et/ou une réalisation d'un geste chirurgical, avertir que le blocage imposé a été forcé, indiquer une valeur des efforts externes appliqués à l'instrument 2 ...

De façon avantageuse, lorsque les moyens de communication indiquent que le blocage imposé a été forcé, le chirurgien est averti que le patient s'est potentiellement déplacé selon un mouvement de grande amplitude et peut ainsi en tenir compte avant de réaliser un geste chirurgical. Dans le cas d'une ponction par exemple, le chirurgien pourra vérifier que l'instrument est bien correctement positionné vis-à-vis de l'organe.

Les moyens de communication comprennent par exemple un écran de visualisation 11.

De préférence, le module de calcul 6 comporte des moyens de mémorisation d'une position à atteindre par l'instrument 2 relativement à l'organe 100. Cette position à atteindre est par exemple entrée par le chirurgien avant le début de l'opération ou bien encore est la position dans laquelle l'instrument se trouvait avant que les moyens de blocage ne soient forcés.

Lorsque le chirurgien déplace l'instrument 2, le module de calcul 6 commande alors les moyens de blocage à partir des images prises par les moyens d'acquisition d'images 10 pour imposer un blocage à au moins l'un des degrés de liberté du bras articulé 3 lorsque l'instrument est sensiblement proche de ladite position.

Il est ainsi possible de bloquer le bras 3 dans une position recherchée précise ce qui facilite le travail du chirurgien.

Le module de calcul 6 commande ici un blocage des moyens de blocage de façon similaire à la façon dont il commande un déblocage des moyens de blocage comme cela a été déjà décrit.

Par exemple, le module de calcul 6 commande un blocage des moyens de blocage selon le procédé de commande suivant :

- acquérir au moins une image de référence de l'organe 100,
- acquérir au moins une première image de l'organe 100,
- calculer un paramètre de similarité entre l'image de référence et la première image,
- comparer le paramètre de similarité à un seuil de similarité prédéterminé qui est représentatif du seuil d'effort prédéterminé,
- commander les moyens de blocage pour bloquer le bras articulé si le paramètre de similarité est supérieur au seuil de similarité prédéterminé.

En variante, le module de calcul 6 commande un blocage des moyens de blocage selon le procédé de commande suivait :

- acquérir au moins une image de référence de l'organe 100,
- acquérir au moins une première image de l'organe 100,
- calculer un paramètre de distance euclidienne et un paramètre de distance angulaire entre l'image de référence et la première image,
- comparer le paramètre de distance euclidienne à un seuil de distance euclidienne prédéterminé et comparer le paramètre de distance angulaire à un seuil de distance angulaire prédéterminé, chacun des seuils étant représentatif du seuil d'effort prédéterminé,
- commander les moyens de blocage pour bloquer le bras articulé si le paramètre de distance euclidienne est inférieur au seuil de distance euclidienne prédéterminé ou si le paramètre de distance angulaire est inférieur au seuil de distance angulaire prédéterminé.

Un mode de réalisation particulier va à présent être décrit. Ce mode de réalisation n'est bien sûr pas limitatif.

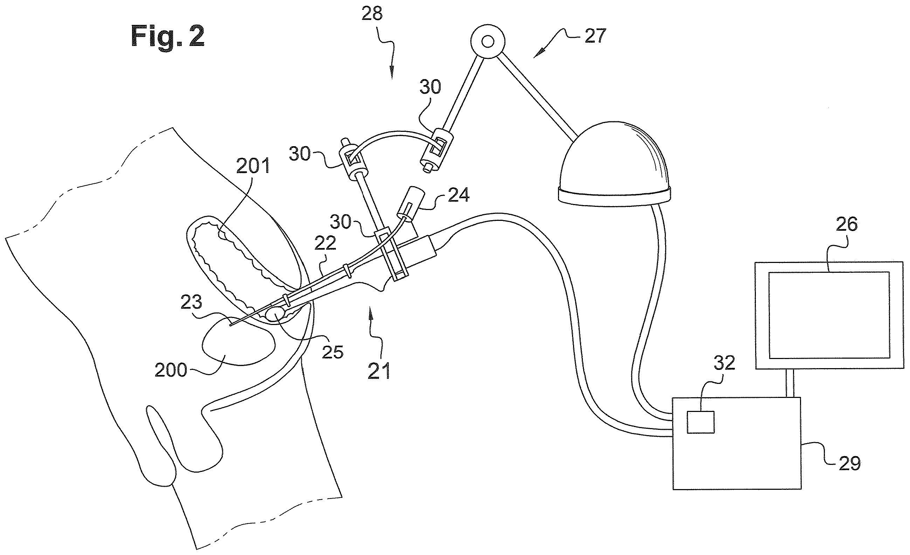

En référence à la figure 2, le dispositif de guidage selon l'invention est ici destiné à approcher un instrument médical 21 à proximité d'une prostate 200 d'un patient. L'instrument médical 21 comporte ici un porte-aiguille 22 qui porte une aiguille à biopsie 23 dont une extrémité est destinée à venir réaliser des ponctions sur la prostate 200. L'instrument 21 comporte en outre des moyens d'actionnement du porte-aiguille 24 pour insérer et retirer l'aiguille 23 de la prostate 200. L'instrument 21 comporte également des moyens d'acquisition d'images de la prostate 200 pour positionner l'aiguille à biopsie 23 relativement à la prostate 200. A cet effet, les moyens d'acquisition d'images comportent une sonde 25. De préférence, la sonde 25 est une sonde échographique permettant d'acquérir des images en trois dimensions de la prostate 200.

Le dispositif de guidage selon l'invention comporte un bras articulé 27 qui comprend une pluralité de degrés de liberté pour déplacer une extrémité proximale de l'instrument 21, c'est-à-dire pour déplacer la sonde 25 et l'aiguille à biopsie 23, dans la voie naturelle qu'est le rectum 201. Ici le bras articulé 27 est conformé pour permettre de déplacer l'extrémité proximale de l'instrument 21 selon au moins un déplacement de l'extrémité proximale de l'instrument 21 selon un axe sensiblement colinéaire à une entrée du rectum 201 lorsque le patient est allongé. Le bras articulé 27 est par exemple un robot Virtuose 3D de la société Haption modifié de sorte que le bras articulé 27 soit lié à l'extrémité proximale de l'instrument 21 par une liaison de type à rotule 28. Bien entendu, le bras articulé 27 est agencé de sorte que la liaison rotule 28 ne puisse influencer un fonctionnement de la sonde 25 ou de l'aiguille à biopsie 23.

De façon privilégiée, le bras articulé 27 est agencé de sorte que la liaison rotule 28 ne puisse empêcher un chirurgien de saisir correctement l'extrémité proximale de l'instrument 21.

Selon l'invention, le dispositif de guidage comporte en outre des moyens de blocage commandés des degrés de liberté du bras articulé 27. Ici, les moyens de blocage sont agencés pour bloquer chaque degré de liberté de la liaison rotule 28 de sorte qu'un blocage imposé à la liaison rotule 28 puisse être forcé lorsqu'un effort externe exercé sur l'instrument 21 dépasse un seuil prédéterminé. A cet effet, les moyens de blocage comportent des freins 30, par exemple des freins à friction, agencés au niveau de chacun des degrés de liberté de la liaison rotule 28. De préférence, le dispositif de guidage est agencé de sorte que lorsque le blocage est forcé, aucune action ne puisse être effectuée sur la prostate 200 par l'aiguille à biopsie 23.

Le dispositif de guidage comporte un module de calcul 29 pour commander ledit bras articulé 27.

Le module de calcul 29 communique avec la sonde 25 pour recevoir et analyser des données transmises par la sonde 25. Des images prises par la sonde 25 sont représentatives de la position de la sonde 25 vis-à-vis de la prostate 200 dans le référentiel du dispositif de guidage. Comme la sonde 25 est à distance et orientation fixes relativement à l'aiguille à biopsie 23 lorsque l'aiguille à biopsie 23 est en position escamotée, ces images sont également représentatives d'une position de l'aiguille à biopsie 23 vis-à-vis de la prostate 200.

Il est donc ainsi possible de corréler le référentiel du dispositif de guidage aux images prises par la sonde 25. Le module de calcul 29 peut ainsi superposer à une image de la prostate 200 prise par la sonde 25, une cible de ponction définie préalablement dans le référentiel du dispositif de guidage. Le module de calcul 29 peut également superposer une trajectoire courante de l'aiguille à biopsie 23 déduite des images prises par la sonde 25 sur une image liée au référentiel du dispositif de guidage qui illustre les précédentes trajectoires de ponction.

Pour commander les moyens de blocage, le module de calcul 29 estime les efforts externes exercés sur l'instrument 21 à partir des images prises par la sonde 25 afin de commander ici un déblocage des moyens de blocage mais également un blocage des moyens de blocage.

Ici, le dispositif de guidage comporte en outre des capteurs agencés sur les différentes articulations du bras articulés 27 de sorte que grâce aux signaux des capteurs, le module de calcul 29 peut estimer la position de l'instrument 21 dans un référentiel du dispositif de guidage.

Le dispositif de guidage comporte ici un écran de visualisation 26 des images prises par la sonde 25 de sorte que le chirurgien puisse avoir un retour visuel et estimer la position de l'aiguille à biopsie 23 relativement à la prostate 200.

De façon privilégiée, le dispositif de guidage comporte différents modes d'utilisation. Selon un premier mode d'utilisation du dispositif de guidage, aucun degré de liberté du bras articulé 27 n'est bloqué. Le bras articulé 27 peut être librement déplacé de façon automatique et/ou de façon manuelle par le chirurgien. Dans un deuxième mode d'utilisation, les degrés de liberté du bras articulé 27 sont bloqués. Le dispositif de guidage comporte en outre des moyens de sélection 32 des modes d'utilisation du dispositif de guidage.

Ici, le module de calcul 29 contrôle également les moyens de blocage du bras articulé 27, et en particulier des freins 30, de sorte qu'il autorise ou non le blocage des degrés de liberté du bras articulé 27 selon le mode d'utilisation du dispositif de guidage sélectionné.

L'acquisition des images avec la sonde 25 et l'analyse de ces images par le module de calcul 29 ne peuvent actuellement être réalisées en temps réel. Grâce au dispositif de guidage selon l'invention, il est possible de maintenir l'instrument 21 dans une position fixe le temps pour la sonde 25 d'acquérir des images de la prostate 200 sans que lesdites images soient déformées du fait d'un mouvement de l'instrument 21 et le temps pour le module de calcul 29 d'analyser ces images pour en déduire une position de l'aiguille à biopsie vis-à-vis de la prostate 200. Ainsi, le chirurgien peut s'assurer que l'aiguille à biopsie est correctement positionnée. En outre, il faut beaucoup de dextérité au chirurgien pour introduire une aiguille à biopsie dans la prostate 200 sans bouger l'instrument. Grâce au dispositif de guidage selon l'invention, il est possible de maintenir l'instrument 2 dans une position fixe durant toute la durée de la ponction.

Ces différentes caractéristiques du dispositif font que l'aiguille 22 est introduite à l'endroit souhaité de la prostate 200 et cela en respectant l'anatomie du patient. Par ailleurs, le dispositif de guidage selon l'invention facilite le travail du chirurgien qui n'a plus à maintenir l'instrument immobile durant toute la durée de la ponction.

Bien entendu l'invention n'est pas limitée au mode de réalisation décrit et on peut y apporter des variantes de réalisation sans sortir du cadre de l'invention tel que défini par les revendications.

## Revendications

1. Système comportant un instrument médical et un dispositif de guidage dudit instrument médical (2; 21) apte à être inséré dans une voie naturelle (101 ;201) ou une voie artificielle d'un patient afin d'amener au moins une extrémité distale (1) de l'instrument à proximité d'un organe interne (100 ;200), le dispositif comportant :

   - un bras articulé (3 ;27) comportant une pluralité de degrés de liberté pour déplacer une extrémité proximale (4) de l'instrument,
   - des moyens de blocage (7 ; 30) commandés des degrés de liberté du bras articulé, les moyens de blocage étant agencés de sorte que, pour au moins l'un des degrés de liberté, un blocage imposé audit degré de liberté puisse être forcé lorsqu'un effort externe exercé sur l'instrument (2 ;21) dépasse un seuil prédéterminé, et
   - un module de calcul (6 ;29) pour commander les moyens de blocage,

   le dispositif de guidage étant **caractérisé en ce qu'**il comporte des moyens d'acquisition d'images (10 ; 25) de l'organe qui sont portés par l'instrument, le module de calcul se basant sur au moins lesdites images pour commander les moyens de blocage.

2. Dispositif de guidage selon la revendication 1, dans lequel le module de calcul (6 ;29) comporte des moyens d'estimation d'un déplacement de l'organe (100 ; 200) relativement à l'instrument (2 ;21) et/ou d'une déformée de l'organe à partir des images fournies par les moyens d'acquisition d'images (10 ; 25), le module de calcul comportant en outre des moyens de contrôle communiquant avec les moyens d'estimation pour déterminer à partir de l'estimation du déplacement et/ou de la déformée si le blocage doit être forcé.

3. Dispositif de guidage selon la revendication 1, dans lequel le module de calcul (6 ; 29) comporte des moyens de mémorisation d'une position à atteindre par l'instrument (2 ;21) relativement à l'organe (100 ; 200), le module de calcul commandant, à partir des images prises par les moyens d'acquisition d'images (10 ;25), les moyens de blocage pour imposer un blocage à au moins l'un des degrés de liberté du bras articulé lorsque l'instrument est sensiblement proche de ladite position.

4. Dispositif de guidage selon la revendication 1, dans lequel les moyens de blocage comportent au moins un moteur (7) agencé sur une articulation du bras articulé (3), le module de calcul (6) commandant les moyens de blocage en modulant un courant électrique traversant le moteur.

5. Dispositif de guidage selon la revendication 1, dans lequel les moyens de blocage comportent au moins un frein (30) agencé sur une articulation du bras articulé (27).

6. Dispositif de guidage selon la revendication 1, dans lequel les moyens de blocage sont agencés de sorte que le seuil prédéterminé soit déterminé en fonction des efforts externes déjà exercés sur l'instrument (2 ;21) lorsque les moyens de blocage (7 ;30) viennent imposer un blocage des degrés de liberté du bras articulé (3 ; 27).

7. Dispositif de guidage selon la revendication 1, comportant des moyens de communication (11 ; 26) pour avertir que le blocage imposé a été forcé.

8. Dispositif de guidage selon la revendication 7, dans lequel les moyens de communication comportent un écran de visualisation (11 ; 26).

9. Dispositif de guidage selon la revendication 1, comportant des moyens de sélection (32) d'un premier mode d'utilisation dans lequel les moyens de blocage sont désactivés de sorte qu'aucun degré de liberté du bras articulé (3 ;27) ne soit bloqué ou d'un deuxième mode d'utilisation dans lequel les moyens de blocage sont activés de sorte que les degrés de liberté du bras articulé (3 ;27) soient bloqués.

10. Dispositif de guidage selon la revendication 1, dans lequel le bras articulé (27) est lié à l'extrémité proximale de l'instrument (21) par une liaison de type à rotule (28).

11. Dispositif de guidage selon la revendication 1, comportant des compensateurs de poids (9) pour équilibrer le bras articulé (3) de sorte que l'extrémité proximale (4) de l'instrument soit maintenue sensiblement au centre de la voie dans laquelle l'extrémité distale (1) de l'instrument (2) est insérée.

**Patentansprüche**

1. System, umfassend ein medizinisches Instrument und eine Führungsvorrichtung zur Führung des genannten medizinischen Instruments (2; 21), das dazu geeignet ist, in einen natürlichen Kanal (101; 201) oder einen künstlichen Kanal eines Patienten eingeführt zu werden, um zumindest ein distales Ende (1) des Instruments in die Nähe eines inneren Organs (100; 200) zu bringen, wobei die Vorrichtung umfasst:

   - einen Gelenkarm (3; 27), der eine Vielzahl von Freiheitsgraden umfasst, um ein proximales Ende (4) des Instruments zu verschieben,
   - gesteuerte Blockiermittel (7; 30) zum Blockieren der Freiheitsgrade des Gelenkarms, wobei die Blockiermittel so ausgebindet sind, dass für mindestens einen der Freiheitsgrade eine dem genannten Freiheitsgrad auferlegte Blockierung erzwungen werden kann, wenn eine auf das Instrument (2; 21) ausgeübte äußere Kraft einen vorgegebenen Schwellwert übersteigt, und
   - ein Rechenmodul, (6; 29) zum Steuern der Blockiermittel,

   wobei die Führungsvorrichtung **dadurch gekennzeichnet ist, dass** sie Bilderfassungsmittel (10; 25) zur Bilderfassung des Organs umfasst, die von dem Instrument getragen werden, wobei sich das Rechenmodul auf mindestens die genannten Bilder stützt, um die Blockiermittel zu steuern.

2. Führungsvorrichtung nach Anspruch 1, bei der das Rechenmodul (6; 29) Schätzmittel zum Schätzen einer Verschiebung des Organs (100; 200) relativ zu dem Instrument (2; 21) und/oder einer Verformung des Organs anhand der von den Bilderfassungsmitteln (10; 25) gelieferten Bilder umfasst, wobei das Rechenmodul ferner Steuermittel umfasst, die mit den Schätzmitteln kommunizieren, um anhand der Schätzung der Verschiebung und/oder der Verformung zu bestimmen, ob die Blockierung erzwungen werden soll.

3. Führungsvorrichtung nach Anspruch 1, bei der das Rechenmodul (6; 29) Speichermittel zum Speichern einer Position umfasst, die von dem Instrument (2; 21) relativ zu dem Organ (100; 200) zu erreichen ist, wobei das Rechenmodul anhand der von den Bilderfassungsmitteln (10; 25) gemachten Bildern die Blockiermittel steuert, um mindestens einem der Freiheitsgrade des Gelenkarms eine Blockierung aufzuerlegen, wenn das Instrument im Wesentlichen nahe der genannten Position ist.

4. Führungsvorrichtung nach Anspruch 1, bei der die Blockiermittel mindestens einen Motor (7) umfassen, der an einer Gelenkverbindung des Gelenkarms (3) angeordnet ist, wobei das Rechenmodul (6) die Blockiermittel steuert, indem es einen durch den Motor hindurchgehenden elektrischen Strom moduliert.

5. Führungsvorrichtung nach Anspruch 1, bei der die Blockiermittel mindestens eine Bremse (30) umfassen, die an einer Gelenkverbindung des Gelenkarmes (27) angeordnet ist.

6. Führungsvorrichtung nach Anspruch 1, bei der die Blockiermittel so ausgebildet sind, dass der vorgegebene Schwellwert in Abhängigkeit von bereits auf das Instrument (2; 21) ausgeübten äußeren Kräften bestimmt wird, wenn die Blockiermittel (7; 30) eine Blockierung der Freiheitsgrade des Gelenkarms (3; 27) auferlegen.

7. Führungsvorrichtung nach Anspruch 1, umfassend Kommunikationsmittel (11; 26), um mitzuteilen, dass die auferlegte Blockierung erzwungen wurde.

8. Führungsvorrichtung nach Anspruch 7, bei der die Kommunikationsmittel einen Anzeigebildschirm (11; 26) umfassen.

9. Führungsvorrichtung nach Anspruch 1, umfassend Auswahlmittel (32) zum Auswählen einer ersten Nutzungsart, in der die Blockiermittel deaktiviert sind, so dass kein Freiheitgrad des Gelenkarms (3; 27) blockiert ist, oder einer zweiten Nutzungsart, in der die Blockiermittel aktiviert sind, so dass die Freiheitsgrade des Gelenkarms (3; 27) blockiert sind.

10. Führungsvorrichtung nach Anspruch 1, bei der der Gelenkarm (27) mit dem proximalen Ende des Instruments (21) über eine Verbindung der Art Kugelgelenk (28) verbunden ist.

11. Führungsvorrichtung nach Anspruch 1, umfassend Gewichtsausgleicher (9) zum Ausgleichen des Gelenkarms (3), so dass das proximale Ende (4) des Instruments im Wesentlichen in der Mitte des Kanals gehalten wird, in den das distale Ende (1) des Instruments (2) eingeführt ist.

**Claims**

1. System comprising a medical instrument and a device for guiding said medical instrument (2; 21) arranged to be inserted into a natural duct (101; 201) or an artificial duct of a patient in order to bring at least a distal end (1) of the instrument in proximity to an internal organ (100; 200), the device comprising:

- an articulated arm (3; 27) with a plurality of degrees of freedom for moving a proximal end (4) of the instrument,
- controlled locking means (7; 30) for restricting the degrees of freedom of the articulated arm, the locking means being designed so that, for at least one of the degrees of freedom, imposed restriction of said degree of freedom can be enforced when an external effort exerted on the instrument (2; 21) exceeds a predetermined threshold, and
- a computing module (6; 29) for controlling the locking means,

the guiding device being **characterized in that** it comprises image acquisition means (10; 25), carried by the instrument, for acquiring images of the organ, the computing module being based on at least said images in order to control the locking means.

2. The guiding device as claimed in claim 1, wherein the computing module (6; 29) comprises means for estimating the displacement of the organ (100; 200) relative to the instrument (2; 21) and/or any deformation of the organ, on the basis of images supplied by the image acquisition means (10; 25), the computing module further comprising monitoring means communicating with the estimating means so as to determine on the basis of the estimated displacement and/or deformation whether locking should be enforced.

3. The guiding device as claimed in claim 1, wherein the computing module (6; 29) comprises means for storing a position to be reached by the instrument (2; 21) relative to the organ (100; 200), the computing module controlling the locking means, on the basis of the images taken by the image acquisition means (10; 25), so as to impose restriction on at least one of the degrees of freedom of the articulated arm when the instrument is substantially close to said position.

4. The guiding device as claimed in claim 1, wherein the locking means comprise at least one motor (7) arranged on an articulation of the articulated arm (3), the computing module (6) controlling the locking means by modulating an electric current passing through the motor.

5. The guiding device as claimed in claim 1, wherein the locking means comprise at least one brake (30) arranged on an articulation of the articulated arm (27).

6. The guiding device as claimed in claim 1, wherein the locking means are designed so that the predetermined threshold is determined according to external efforts already exerted on the instrument (2; 21) when the locking means (7; 30) impose restriction on the degrees of freedom of the articulated arm (3; 27).

7. The guiding device as claimed in claim 1, comprising communication means (11; 26) for warning that imposed locking has been enforced.

8. The guiding device as claimed in claim 7, wherein the communication means comprise a display screen (11; 26).

9. The guiding device as claimed in claim 1, comprising means (32) for selecting a first operating mode wherein the locking means are deactivated so that no degree of freedom of the articulated arm (3; 27) is restricted or a second operating mode wherein the locking means are activated so that the degrees of freedom of the articulated arm (3; 27) are restricted.

10. The guiding device as claimed in claim 1, wherein the articulated arm (27) is linked to the proximal end of the instrument (21) by a ball-joint type link (28).

11. The guiding device as claimed in claim 1, comprising weight balancers (9) to balance the articulated arm (3) so that the proximal end (4) of the instrument is held substantially in the center of the duct into which the distal end (1) of the instrument (2) is inserted.

# Fig. 1

**Fig. 2**

EP 2 760 361 B1

Départ

Acquisition
$I_r$

Transmission
$I_r$

101

Séquence
d'initialisation

Départ

Acquisition
$I_s$

102

Transmission
$I_s$

103

Calcul
S

Séquence
de déplacement

104

Comparaison
S et $S_{min}$

105

Déblocage

Fig. 3

```
                    ┌─────────────────────────┐
                    │        ( Départ )        │
                    │           │              │
                    │           ▼              │
                    │    ┌────────────┐        │
201 ⌇               │    │ Acquisition│        │        Séquence
                    │    │    I_r     │        │        d'initialisation
                    │    └────────────┘        │
                    │           │              │
                    │           ▼              │
                    │    ┌────────────┐        │
                    │    │Transmission│        │
                    │    │    I_r     │        │
                    │    └────────────┘        │
                    └───────────┼─────────────┘
```

Départ

Acquisition $I_r$

Transmission $I_r$

Séquence d'initialisation

Départ

Acquisition $I_s$

202

Transmission $I_s$

203 — Calcul $D_e$ et $D_a$

Séquence de déplacement

204 — Comparaison $D_e$ et $D_{e\ min}$ et $D_a$ et $D_{a\ min}$

205 — Déblocage

# Fig. 4

**Fig. 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011058530 A